# EUROPEAN PATENT APPLICATION

(11) **EP 3 108 920 A1**
(43) Date of publication of application: **28.12.2016**
(21) Application number: 15173171.8
(22) Date of filing: 22.06.2015
(51) Int. Cl.: A61M 16/12, A61M 16/20, A61M 16/00, A61M 16/10

(54) **DEVICE FOR DELIVERING NITRIC OXIDE AND OXYGEN TO A PATIENT**

(71) Applicant: Linde AG, 80331 München (DE)
(72) Inventor: JAFRI, Syed, LONDON (GB); FIEDLER, Konstantin, 81675 Munich (DE)
(74) Representative: Nordmeyer, Philipp Werner

(57) **Abstract**

The present invention pertains to a device (1) for the treatment of respiratory disorders or complications thereof in a mammal with a gaseous mixture for use as an inhalable medicament, comprising a patient interface (3), a source (23) of air, a source (21) of gaseous nitric oxide, a source (22) of gaseous oxygen, an application device (4) for providing a gaseous mixture to a patient interface, at least one gas injector (5) for injecting nitric oxide provided by the source of gaseous nitric oxide into the gaseous mixture provided by the application device, at least one gas injector (6) for injecting oxygen provided by the source of gaseous oxygen into the gaseous mixture provided by the application device, and a controller (8) programmed for controlling the at least one gas injector and the application device, wherein the source of gaseous nitric oxide comprises an arrangement (210) for onsite production of nitric oxide, and the source of gaseous oxygen comprises an arrangement (220) for onsite enrichment of oxygen.

## Description

### Technical Field

The invention relates to a device for the application of a gaseous mixture as an inhalable medicament to a patient.

### Technological Background

Patients with respiratory disorders often suffer from pulmonary luminal obstructions that impair breathing. The pathophysiological development of such disorders can cause fluid build-up in the lungs, leading to reduced gas exchange due to pulmonary shunting. In cases where the patient is unable to adequately clear these fluids, the concomitant obstructions in e.g. bronchioles and alveoli further reduces the efficacy of pulmonary gas exchange leading to insufficient blood oxygenation and blood acidification due to insufficient carbon dioxide expiration.

Such obstructions not only result in limited gas exchange but may also cause a coughing reflex or induce breathing spasms to improve clearance. However, for patients that are incapable to perform clearance, e.g. due to a reduced production of surfactant, in case of chronic diseases, or when the amount of fluid is too high for natural clearance, such pulmonary activation leads to irregular contractions. This further increases the patient's burden since this not only increase the patient's fatigue level and psychological distress, but also results in impaired pulmonary gas flow caused by unwanted vortices and inadequate ventilation due to suboptimal breathing patterns. As a result, blood oxygenation is further reduced, leading to even more severe symptoms. This negative loop may in severe cases eventually lead to a collapse of the patient, requiring medical intervention.

The insufficient fluid clearance together with the altered pathophysiological conditions allows the intrusion of pulmonary pathogens, which may lead to pulmonary infections such as pneumonia. Pneumonia is an inflammatory condition of the lung primarily affecting the alveoli resulting from infection with bacteria and/or viruses, less commonly by other organisms such as fungi or parasites. Bacteria generally enter the upper respiratory tract through aspiration of small quantities of microbial cells present in the nose or throat (particularly during sleep), via airborne droplets, or through gastric reflux. Systemic sepsis or septicaemia may also result in bacterial invasion of the lungs. Viral infection may occur through inhalation or distribution from the blood; the lungs cells lining the airways, alveoli, and parenchyma are damaged, and may render the patient more susceptible to bacterial infection of the respiratory tract.

In case of e.g. microbes such as bacteria, their rapid growth causes a change in the microenvironment, leading to acidification and cell death in pulmonary tissue. When pulmonary shunts exist, this not only reduces the blood oxygenation, but also leads to a blood flow reduction. The normally occurring foreign body reaction is hence impaired since the inflammation induced mobilization and extravasation of host defence cells such as macrophages, leukocytes, natural killer cells, and/or mast cells to engulf and inactivate invading bacteria cannot sufficiently target the growth of pathogens. The reduced blood flow and clearance in addition lead to a build-up of necrotic and apoptotic cells, resulting in pus accumulation. Furthermore, the concomitant fluid extravasation into the alveoli from surrounding blood vessels may even worsen existing pulmonary shunts, further impairs breathing efficiency, restricts influx of respiratory gas to the affected alveoli thereby reducing gas exchange efficiency, and particularly damages the affected lower respiratory tract.

A further problem with bacterial growth is their secretion of macromolecules that provide an optimal microenvironment for proliferation. The accumulation of these macromolecules leads to the development of a so-called biofilm. Known to be a major cause for the gradual intolerance of medical implants, such biofilms are difficult to eliminate. The environment provided for these pathogens enables their colonization in a region that is difficult for the host's immune system to infiltrate or impairs induced extravasation, causing the readily formation of biofilms within 12 hours. Since bacteria preferably grow within the biofilm, known bactericidal agents such as antibiotics are mostly ineffective since they cannot penetrate and/or diffuse through the biofilm in sufficient concentrations to cause bacterial cell death. Ineffective clearance of such a biofilm may cause further accumulation and eventually leads to droplet formation and spreading of the pathogen through the respiratory system.

Reduced humidification, a reduced cough reflex and the impaired extravasation and infiltration of the immune system into the pathogenic area render the host's defense mechanism incapable of proper clearance. Prevention and treatment of these complications and symptoms are therefore of key interest for increasing survival.

Especially in the situation where pulmonary infections are associated with respiratory disorders, as described above, a systemic approach to target pulmonary infections, e.g. by ingestion and distribution through blood circulation of an antibiotic, would provide low efficiency since i) blood flow at the desired target site is often reduced due to shunting, ii) the delivery of the therapeutic is ineffective since the target site is not at a systemic location, and iii) the low permeability of the biofilm does not provide sufficient diffusion of the therapeutic agent.

Hence, the problem with conventional therapies is that antibiotics are insufficiently directed to target sites and furthermore require high doses to provide a desired bactericidal effect. When bacterial growth is not properly targeted or the therapeutic effect is not readily achieved, this furthermore forms the risk of inducing bacterial resistance to these therapeutic agents, providing an even larger burden on the patient. These high doses in addition may cause unwanted and severe side effects, leading to e.g. ingestion and/or digestion problems, skin irritation and inflammation, dehydration, nausea etc.

For obvious reasons, opposed to e.g. dermatological disinfection, pulmonary infections cannot be treated through elimination with alcohol incubation and other conventional methods that should prevent or reduce the risk of pneumonia or reduce the signs and symptoms thereof, such as frequent exogenous aspiration of secretions, prophylactic administration of antibiotics or saline lavage, in fact aggravate the patient's condition.

Although the application of oxygen alleviates the patient's condition by providing improved oxygen intake and gas exchange, thereby also reducing the patient's sensation of insufficient breathing and accompanying distress, this does not treat the underlying cause of impaired respiration. An alternative approach therefore includes the application of nitric oxide, potentially in combination with the application under positive airway pressure. Nitric oxide (NO) has many known biological functions. Ranging from neurotransmission, cellular differentiation to regulation of cellular oxygen consumption through effects on mitochondrial respiration, it also regulates the host immune response by e.g. inhibition of leukocyte adhesion and regulation of NFκB levels *in vivo.* Its use in treatment of diseases affecting the respiratory tract, however, bases on the relaxation of smooth muscle cells lining the vascular system.

Endogenously induced NO oxidizes the iron atom of a haem moiety in the enzyme soluble guanylate cyclase (SGC) in the smooth muscle cells of the lower respiratory tract airways, in the pulmonary arteries and in the membranes of circulatory platelets, thereby activating the SGC. The activated SGC forms the second messenger cGMP, which in smooth muscle cells promotes calcium-dependent relaxation, causing vasodilation of blood vessels in the lower respiratory tract, thereby increasing blood flow through the pulmonary arteries and capillaries, and also dilation of the airways in the lower respiratory tract, thereby improving bulk gas transport into the alveoli and exchange of O₂ and CO₂. Hence, administration of NO leads to a reduction of local blood pressure, stimulates vasodilatation and thereby facilitates gas exchange in the alveoli of the lungs. A further result is reduction of platelet aggregation on irregular surfaces (such as a constricted blood vessel) thereby lowering the probability of thrombosis (see e.g. WO 95 / 10315 A1).

Biologically produced NO is synthesized *in vivo* by both constitutive and inducible isozymes of the nitric oxide synthases (NOS), which catabolize L-arginine to NO and citrulline. Endothelial constitutive NOS (eNOS), present in the walls of bronchioles and pulmonary arterioles provide NO at nanomolar concentrations for regulating vessel tone.

Inhalable gaseous NO (IgNO) may be used to relax smooth muscle control of pulmonary arteriole diameter, for treating pulmonary hypertension in diseases such as acute respiratory distress syndrome (ARDS), in which impaired gas exchange and systemic release of inflammatory mediators ('acute phase proteins' and cytokines, particularly interleukins) cause fever and localised or systemic increases in blood pressure. IgNO will also relax smooth muscle control of bronchiole diameter, for treating emphysema in cases of ARDS and chronic obstructive pulmonary disease (COPD), in which the lower respiratory tract (particularly the lung parenchyma: alveoli and bronchioles) become inflamed. In COPD airways in the lower respiratory tract narrow and lung tissue breaks down, with associated loss of airflow and lung function which is not responsive to standard bronchodilating medication. IgNO administration may therefore assist in countering the 'pulmonary shunt', in which respiratory disease causes deregulation of the matching of the flow of air to the alveoli with the blood flow to the capillaries, which under normal conditions allows oxygen and carbon dioxide to diffuse evenly between blood and air (see e.g. WO 95 / 10315 A1).

Furthermore, endogenously produced NO is partially responsible for the cytotoxic actions of macrophages due to its cell damaging activities. IgNO therefore has another advantage by providing an effective microbicidal molecule for treating infections of the respiratory tract that acts directly *in situ,* whereas parenteral administration of drugs requires a high dosage to address systemic dilution and hepatic catabolism. Thus, NO has been shown to be an effective agent for killing *Mycobacterium tuberculosis* within cysts or tuberculi in a patient's lungs (see WO 00 / 30659 A1). IgNO may also be administered to treat pneumonia: pulmonary infection and inflammation (see WO 00 / 30659 A1).

Isozymes of inducible NOS (iNOS) are present in many cell types; upon activation they temporarily produce NO at micromolar concentrations, an activity which, under pathological conditions, has been associated with production of superoxides, peroxynitrites, e.g. upon reperfusion injury of ischemic tissue, inflammation and cellular damage. Hence, the application of NO has many negative side effects and can be highly toxic if applied in high dose for prolonged periods of time. The high reactivity of NO in pure form causes limited solubility in aqueous solutions. Consequently, delivery of NO is typically performed by administration of a prodrug which is metabolically degraded, or through direct inhalation of gaseous NO (IgNO).

The toxicity of IgNO is associated with a variety of properties.
(a) Firstly, NO is swiftly absorbed by lung tissue and enters the blood stream, where it reacts very rapidly with haemoglobin, oxidizing the iron atom of one of the four haem moieties to the ferric form, thereby creating stable methaemoglobin (+ nitrite and nitrate ions), inhibiting electron transport pathways and energy metabolism. Methaemoglobin's three ferrous haem groups have far greater affinity for oxygen than the haemoglobin haem moieties, so that blood in which the proportion of methaemoglobin is elevated releases insufficient oxygen to the tissues.
(b) Secondly, in the presence of oxygen NO reacts rapidly to form nitrogen dioxide (NO₂), itself a toxic molecule and forming acidic compounds in aqueous environments. Gaseous NO₂ at 5 ppm is considered to be a toxic concentration, compared to standard administrations of IgNO at between 10 to 40, maximum up to 80 ppm. As lung disease frequently causes reduced respiratory function, patients are often administered an O₂-enriched air supply. In the presence of such an increased concentration of O₂ the probability of NO being oxidized to toxic NO₂ is correspondingly greater.
(c) Thirdly, NO reacts with superoxides, increased in many disease states due to oxidative stress, to form toxic peroxynitrites, powerful oxidants capable of oxidizing lipoproteins and responsible, as are both NO and NO₂, for nitration of tyrosine residues. Peroxynitrite reacts nucleophilically with carbon dioxide, which is present at about 1 mM concentrations in physiological tissues, to form the nitrosoperoxycarbonate radical. This, in turn, degrades to form carbonate radical and NO₂, both of which are believed to be responsible for causing peroxynitrite-related cellular damage. Nitrotyrosine is used as an indicator of NO-dependent nitrative stress induced in many disease states, generally being absent or undetected in healthy subjects.
(d) Fourthly, NO₂ oxygenizes cobalt in cobalamin (vitamin B12), leading to a loss of serum methionine, responsible for the conversion of uridine to the nucleotide thymidine. The reduced availability leads to a loss of DNA production and/or repair and results in impaired cell division, cell-cycle arrest, and/or induced apoptosis.

For both intermittent and continuous application of NO and oxygen at tolerable doses to patients, sufficient resources are required to be present during treatment. Since patients often require application of NO and oxygen for long-term treatment and for prolonged periods of time, large amounts of NO and oxygen need to be provided. As a source of NO and oxygen, large bottle-shaped tanks are therefore commonly used, which not only provide transport and storage difficulties, but are also cost-intensive and may be inconvenient and/or difficult to handle. For medium to large scale medical institutions and clinics this furthermore requires sufficient logistical and room planning to reduce the occurrence of insufficient capacity and hence ensure that patient treatment is not impaired. Thus, for cases where sudden changes in demand arise, either expected or unexpectedly, a buffer in capacity is required to increase flexibility and reduce potential waiting times. This further increases the costs at the expense of treatment efficiency.

Onsite nitric oxide production methods are known in the art. US 5,396,882, WO 2013/052548 A2, and WO 2014/143842 A1 disclose e.g. the application of an electric arc or plasma to enrich NO from air, whereas US 2006/172018 provides a method for obtaining NO by controlling the diffusion and/or dissolution of nitride salts or other precursor compositions. NO can also be derived through a reaction with reduction agents. An example of such a method can e.g. be found in US 2011 /220103.

However, onsite production of nitric oxide, in particular in case of patients with long-term treatment requirements, requires the respective patient to stay at the medical facility long-term or at least for with frequent visits for prolonged periods of time. The flexibility of the patient's treatment hence is not improved.

Accordingly, a need exists to improve flexibility of NO and oxygen treatment, preferably while improving safety management and/or reducing safety issues.

### Summary of the invention

It is an object of the present invention to provide a device for the treatment of respiratory disorders or complications thereof in a mammal, e.g. a human patient, with a gaseous mixture for use as an inhalable medicament.

In a first aspect, a device is suggested, which comprises at least a patient interface, a source of air, a source of gaseous nitric oxide, a source of gaseous oxygen, and an application device for providing a gaseous mixture to a patient interface. For injecting nitric oxide, provided by the source of gaseous nitric oxide, into the gaseous mixture provided by the application device, it furthermore comprises at least one gas injector. By the same token, the device comprises at least one gas injector for injecting oxygen provided by the source of gaseous oxygen into the gaseous mixture provided by the application device. The device may further comprise a controller programmed for controlling the at least one gas injector and the application device. The source of gaseous nitric oxide may further comprise an arrangement for onsite production of nitric oxide and the source of gaseous oxygen may further comprise an arrangement for onsite enrichment of oxygen.

The source of air may be provided by ambient air. Preferably, the source of air comprises a filter arrangement for providing air without pollutants. Accordingly, potential toxic chemical compounds such as carbon monoxide, ozone, sulfur dioxide, dust, and/or particulate matter, may be filtered out. In addition, the filter arrangement may be configured to prevent pathogens to enter the device and consequently enter the respiratory system of the patient. Pathogens such as e.g. viruses and/or airborne bacteria may hence be filtered out. Accordingly, the source of air may comprise a filter arrangement for providing sterile medical grade air. Filters with a variety of mechanisms may be provided, e.g., chemical-based, mechanical, ionic binding-based, absorption-based, electromagnetic, etc. In addition, the filter arrangement may alter the humidity level of the ambient or sterile medical grade air before it enters the device, in particular the application device. To ensure appropriate flow to the application device, the source of air may furthermore comprise a pumping device or compressor. Alternatively, the source of air may be integrated into the application device.

The onsite nitric oxide production may be provided and generated in-line. NO may then be mixed into the gaseous mixture at a gas injector. Such methods and apparatuses are known in the field and allow the generation of NO through nitrogen and oxygen present in ambient air by for example a pulsating electrical discharge or an electric arc, see e.g. WO 2013/05248 A2 and WO 2014/143842 A1, respectively. The methods described here should not be appreciated such that these are limiting, but merely provide examples from a plurality of alternative methods known in the art.

Onsite production of nitric oxide and onsite enrichment of oxygen at least has the advantage that nitric oxide and oxygen can be made readily available for patient treatment. This not only reduces the risk of potential deleterious side reactions due to prolonged storage time, but also ensures that required concentrations of e.g. nitric oxide may be provided whenever demand exists.

Furthermore, the onsite production of nitric oxide and the enrichment of oxygen greatly reduce the need for storage facilities, planning, and the costs associated therewith. In addition, since medium to large scale medical facilities and clinics often provide medical grade gases through central supply conduits, e.g., pipes and/or tubes integrated in the facility's walls, maintenance and servicing may be cumbersome and often require a temporary supply outage. During these supply disruptions, back-up systems may not be available and relevant parts may furthermore not be easily exchangeable leading to potential impaired treatment. Onsite production of nitric oxide and onsite enrichment of oxygen according to the invention does not require central supply systems and eliminates the problems associated therewith.

Due to the onsite production of nitric oxide and the enrichment of oxygen, transportation and logistical planning of large-weight supply tanks, including the costs associated therewith, are avoided. Accordingly, the device may function as an autonomous device that may be installed at any location other than a medical facility. This is in particular advantageous for patients that require long-term and/or frequent application with the gaseous mixture while e.g. requiring other treatments or medical care at home. Furthermore, the patient may not be capable of frequently visiting a medical facility or only with large physical effort. Hence, the device may also be installed at a patient's home to provide home care. In addition, home care not only allows the patient to be treated in a more comfortable, personal environment, but also allows a more flexible treatment since it is no longer limited to available appointments with a medical professional. By the same token, the costs and time associated with medical care for the patient may be greatly reduced. Home care, as provided with the device according to the invention, allows a patient to engage in both social and physical activities and furthermore may provide better resting and/or recovering opportunities, in particular during night time. Quality of life of the patient is hence improved.

According to a further aspect of the invention, the device components are provided within a single housing. A single housing not only increases accessibility for a medical professional to e.g. inspect, install, initiate, control, or adjust the device, but also optimizes space occupation, facilitates cleaning and hygiene management, reduces the risk of losing and/or damaging otherwise external components, facilitates identification and transportation, and facilitates patient treatment. In case of defect components, the housing may be easily identified and repair and/or exchange of components may hence be faster.

Aside from the disadvantage of common technologies that the application of a gaseous mixture is medical facility bound and/or restricted is the reduced mobility of the patient. Hence, according to another aspect of the invention, the device is configured to be portable. With the term portable, any configuration is meant that allows the device to be displaced, moved, and/or transported to any other location or at least wherein the device is not fixed or bound to a single location. Preferably, the device may be configured to be manually carried, i.e. the device may be configured as, for example, a trolley, a stand with rollers, a case, or any other device, preferably hands-free, such as, for example, a backpack. The configuration as a portable device at least has the advantage of increasing the mobility of the patient by enabling treatment of the patient at a location other than a clinic. For example, the device may be placed in a patient's home so that driving to the hospital or clinic may no longer be necessary or, alternatively, the patient may carry the device on his/her back, when configured as a portable device similar to a backpack, preferably in a single housing, so that the patient may engage in physical activities such as e.g. walking, or in cases wherein only intermittent treatment is required also other and/or more demanding sports or sports wherein increased movability is required. Thus, a device configured to be portable may provide on-demand supply of oxygen and nitric oxide and facilitates increased mobility of a patient. Preferably, the device is furthermore configured to avoid the often complex mixture of, e.g., devices, tubing, and wiring in common technologies, thereby reducing any movement restrictions associated therewith. According to another aspect of the invention, the device may comprise at least one rechargeable electrical energy storage device and may be configured to operate as a stand-alone device. Preferably, rechargeable electrical energy storage devices may be chosen that are known in the art such as, e.g., rechargeable batteries, in particular lithium-ion batteries, capacitors, or electrical energy storage devices based on, e.g., kinetics, photovoltaics, or other environmentally friendly techniques. By implementation of a rechargeable electrical energy storage device the device may operate autonomously and independent of its location. The electrical energy storage device may also be configured as a hybrid, e.g., a combination of a kinetic energy storage device and a rechargeable battery, to provide both a short-term and long-term energy supply, and/or to provide a back-up energy device. In particular, the implementation of e.g. a rechargeable battery is beneficial when configuring the device as a portable device, wherein preferably the components are provided within a single housing. Such a configuration provides the patient to be treated with increased mobility and flexibility. However, if the device is installed e.g. at a patient's home, the device may also be connectable with a central electricity supply system, e.g. through connection to a wall socket. The rechargeable electrical energy storage device may then function as e.g. a back-up system for temporary instances of power outage to ensure continued functioning of the device.

The device may further comprise a connecting means for charging of the electrical energy storage device. Furthermore, the device may comprise a battery indicator and/or alarm to indicate to a user that recharging of the electrical energy storage device is required. Settings and/or thresholds for such an alarm may be variable and/or user adjusted, depending on, e.g., mobility, time, or urgency of treatment.

According to another aspect of the invention, the source of gaseous oxygen may comprise a reservoir for storing gaseous oxygen. In this aspect the reservoir is configured to be in fluid communication with the source of gaseous oxygen. The implementation of a reservoir at least provides the advantage that small volumes and/or concentrated volumes of the respective gas may be stored, which may provide an increased flow rate, i.e. respiration rate, of the respective gas, when e.g. a spontaneous increase of demand arises in an emergency situation. Furthermore, the onsite production and/or enrichment may produce, e.g., unwanted vibrations, heat production, and/or noise. Storage of the respective gas in a reservoir may, for example, allow a user to fill the reservoir with a sufficient volume or at least a volume sufficient for initial treatment before using the device at a later time point. To further increase the capacity of the device, a compressor may be provided to provide the reservoir with a compressed volume of the respective gas. The at least one reservoir may furthermore comprise a combination of gases, e.g. sterile air enriched with oxygen. This may furthermore allow a patient to use the device in a plurality of situations, wherein the use of electrical devices is prohibited or limited, for example, at airports or when flying. The device may hence also be configured to have an airplane mode or the like, known in the art.

Accordingly, the device may also comprise a sensor arrangement for detecting the volume and/or the concentration of at least one of the stored gaseous nitric oxide or stored gaseous oxygen. This not only increases the safety of applying the appropriate concentrations of the gaseous mixture, but also allows the user or medical professional to inspect and potentially adjust the storage level and storage efficiency.

The device may further comprise a filter and/or a reservoir for exhaled breathing gases. A filter may filter, collect, and/or transfer exhaled breathing gases to prevent dissipation of the gaseous mixture or of e.g. carbon dioxide or nitric oxide into ambient air. This not only reduces potential contamination of exhaled air with the to be inhaled gaseous mixture, which may provide unwanted gas concentrations to be inhaled and/or an inefficient respiration, but also provides increased safety for surrounding patients, medical personnel and the environment by preventing introduction and accumulation of potential toxic compounds or toxic levels in the surrounding air. Alternatively, or in addition, a filter may also adsorb e.g. humidity for e.g. acclimatizing reasons or to reduce potential detrimental side effects with device components. Preferably, exchangeable filters are chosen for ease of maintenance. The reservoir may provide e.g. storage of gases until they are disposed of or for recycling purposes. A filter may also be provided before entry into the reservoir.

In addition or alternatively, the exhaled breathing gases may be in communication with the arrangement for onsite production of nitric oxide and/or with the arrangement for onsite enrichment of oxygen. Since e.g. nitric oxide is normally not fully absorbed after application, re-use of this compound may increase the efficiency of the device to provide sufficient nitric oxide. Accordingly, a filter arrangement may be implemented to provide recycling of the exhaled breathing gas or parts thereof for further application.

According to another aspect of the invention, the controller may comprise at least one processing means for processing of an algorithm to cause a target cumulative dose of the gaseous mixture to be applied by the at least one gas injector and the application device. Preferably, the target cumulative dose of the gaseous mixture may be defined by a target cumulative dose of nitric oxide.

Since the application of gaseous mixtures other than ambient air introduces a physiological change in the respiratory system of a patient to be treated, toxicity of the gaseous mixture and hence the application thereof needs to be monitored. The algorithm may therefore be programmed to evaluate different variables such as, e.g., a pre-set patient-specific (total daily) dose of the gaseous mixture to be applied, a desired dose to be gradually applied and limits at which the gaseous mixture is to be applied, the actual applied dose and/or the actual cumulative dose applied, the duration of the application of the gaseous mixture, the time of day, etc.

The device may also comprise a sensor arrangement for the detection of the concentration of at least one component of the gaseous mixture and which is in communication with the controller. Accordingly, the controller may be provided with a feedback mechanism from e.g. a gas sensor to detect, e.g., the concentration of a gas after enrichment and/or production of the respective gas or detect the concentration of a gas in the gaseous mixture before application, preferably upstream of the application device. The controller may accordingly adjust the gas injection and/or the application of the gaseous mixture. Depending on the configuration of the device, the sensor arrangement may also provide feedback to the algorithm, e.g. as an input of a variable.

Likewise, the device may comprise a sensor arrangement for the detection of the concentration of at least one component of exhaled breathing gases which is in communication with the controller. Such a detection provides information about the efficacy of the application of the gaseous mixture and gas exchange and furthermore may increase safety of the patient and the treatment since potential physiological changes in the patient and the presence of toxic levels and/or compounds may be recognized. If, for example, the nitric oxide concentration in the exhaled breathing gas shows a sudden increase, application of the gaseous mixture may be altered and/or a medical professional may be notified, e.g. by an alarm. Alternatively, the presence of molecules due to unwanted side reactions, e.g. nitrogen dioxide, may also alert a medical professional. If applicable, and preferably, the sensor arrangement may also provide feedback to the algorithm, e.g. as an input of a variable.

In addition, the sensor arrangement for the detection of the concentration of at least one component of exhaled breathing gases may comprise a sensor for detecting a change in the physiological state of the patient. For example, the exhaled breathing gases can furthermore be used to measure concentrations of e.g. metabolic waste products such as carrier compounds, nitrogen, ammonia, or lactates. These measurements may provide information about e.g. microbial growth, drug absorption and/or metabolism. Hence, according to another embodiment of the present invention, the device comprises means to measure molecular concentrations present in the exhaled breathing gas. Such means are known in the art, such as e.g. chemical sensors. The sensor arrangement may accordingly provide feedback to the controller and/or the algorithm regarding efficacy of the application and efficiency of the breathing of the gaseous mixture.

Furthermore, the controller may be programmed to initiate the application of the gaseous mixture automatically or manually. For example, the controller may automatically initiate the application via the gas injector and the application device according to a pre-set application regimen. Alternatively, the controller may initiate the application of the gaseous mixture upon an action of the user or medical professional, e.g. by pressing of a button. In this case, the patient may receive on-demand application of an appropriate gaseous mixture.

Furthermore, the device may be programmed for different, preferably patient-specific, treatment regimens. For example, a treatment regimen may provide e.g. a nitric oxide delivery peak in the morning to cleanse the lungs after sleeping and/or before the patient falls asleep in the evening.

Alternatively, or in addition, the target cumulative dose may vary between 24 hour periods, e.g., allowing for days off therapy or intensive therapy, when the patient, e.g., copes with an infection, or when treatment may cause deteriorating symptoms in the patient. Furthermore, the target cumulative dose may vary as part of a multiday treatment schedule.

Furthermore, the controller may be programmed to cause the gaseous mixture to be provided continuously, intermittently, and/or at a predetermined time interval. Accordingly, the target cumulative dose, e.g. of nitric oxide, may also be applied by varying the application within a 24 hour period, between days, and/or weeks. For example, a static dose level may be continuously applied to provide a form of background therapy or prophylaxis at a continuous low dose. Alternatively, intermittent application may be preferred, so that the device applies the gaseous mixture at a static dose level at different time points, for example, as part of a resistant bacteria eradication protocol, or for shorter durations, e.g. for more active patients receiving oxygen co-therapy. When providing intermittent application, the patient may breathe non-enriched, preferably sterile, air provided by the device or, alternatively, may breathe ambient air during recovery periods. However, for both intermittent and continuous application the target cumulative dose preferably does not change. When applying the gaseous mixture for larger time periods, the target cumulative dose may be varied on e.g. a daily or weekly base. For example, nitric oxide may be applied during a first week of treatment while being absent or being present at a lower dose in the gaseous mixture during application in subsequent weeks. This allows weaning of a patient, i.e. gradual reduction of the treatment and hence a gradual increase in autonomous breathing of the patient while preventing e.g. the occurrence of a respiratory infection or a relapse thereof. This is in particular beneficial for patients suffering from COPD.

The application of the gaseous mixture may also occur as a combination of a continuous and an intermittent application. For example, a continuous low dose of nitric oxide may be applied to a patient while a higher dose of nitric oxide may be intermittently applied. The intermittent application may be provided by multiple short high dose bursts lasting e.g. for 10 seconds up to 30 minutes, but may also last longer, e.g. be varied on a daily basis, as described above. The duration time of these bursts may be dependent on the therapeutic goal to be achieved, e.g., minimize coughing, respiratory irritation, and other symptoms. Preferable, the time function may be adjustable according to the algorithm and may hence vary not only per patient but also with an altered physiological state of the patient. The combination of continuous and intermittent application may be particularly beneficial over night, when symptoms that may produce sleep disturbance are to be reduced.

The application at a given or calculated time interval may be in particular beneficial for patients and therapies, wherein the gas inhalation is performed over prolonged periods of time and frequently needs to be interrupted for e.g. further therapeutic treatment, personal hygiene measures, or nourishment. During periods in which no gaseous mixture is applied, the patient can then breathe ambient air.

When applying intermittent dosing, not only shorter treatment durations may be chosen, but the dose to be applied may also be higher, when compared with e.g. continuous application or background therapy, as described above. Application of a high dose of e.g. nitric oxide in the evening before bed time may expectorate secretions, cause microbial kill, and/or suppress overnight bacterial regrowth. Simultaneously, application of a high dose of e.g. nitric oxide in the morning expectorates secretions/microbes that have accumulated overnight so that the microbial and/or secretion load is reduced throughout the day, enabling e.g. more effective respiratory physiotherapy, higher activity, and/or improved delivery of inhaled medicaments for the patient. The high and low doses of the gaseous mixture may also be combined with different treatment regimens, as, for example, described above.

According to another aspect of the invention, the device may comprise a flow rate sensor which is in communication with the controller. The flow rate sensor may detect a flow change or an absolute flow rate in e.g. a conduit of the application device to the patient interface and may hence derive or determine a breathing phase. The measured value or change is then provided as an input to the controller to, e.g., determine and/or adjust the application of the gaseous mixture. Preferably, the at least one flow rate sensor and/or the controller may be in communication with the processing means to provide input to the algorithm. Using this input, the algorithm may determine, e.g., breathing patterns, breathing intervals, breathing volumes, breathing deficiencies, etc., over time and may accordingly adjust the application of the gaseous mixture and/or the application pattern during a period of time, e.g. 24 hours, to provide an optimal patient-specific application of the target cumulative dose. Furthermore, when applying the dose intermittently, the flow rate sensor may also provide similar breathing information from phases, wherein no nitric oxide and/or oxygen into the gaseous mixture are injected. The algorithm may accordingly adjust the application pattern or treatment regimen of the gaseous mixture.

The controller may further be programmed to apply the gaseous mixture with a breath by breath variability and/or with a predetermined breathing frequency. The application may hence also be provided with e.g. a pre-programmed breathing frequency, which is independent of the measurements provided by a flow rate sensor. For example, the patient may receive the application with a prescribed breathing frequency and may be, actively or passively, required to adjust his/her breathing frequency accordingly. Alternatively, or additionally, the application may be provided variable breath by breath, e.g., the controller may be programmed to apply the gaseous mixture with a prescribed variability per breath or may provide the application of the gaseous mixture in an on-demand fashion, as described above. Preferably, the controller is provided with input from a flow rate sensor and/or from an output of an algorithm to accordingly provide a breath by breath variability and/or a predetermined breathing frequency.

According to another aspect of the invention, the controller may be programmed to control the application of the gaseous mixture in a constant concentration throughout the inhalation cycle, in at least a pulse throughout an inhalation cycle, during every second breathing cycle or during every other natural number of breathing cycles except one. For example, application of the gaseous mixture may be more beneficial during a specific breathing phase, e.g. at the beginning of the inspiratory breath for optimal spreading throughout the respiratory system, or when bracheoli and alveoli of the respiratory system are in their most accessible or widened state, so that e.g. a higher dose of the gaseous mixture may be chosen when applying the gaseous mixture in at least a pulse throughout the respective phase. This at least has the advantage of increasing efficacy of the gaseous mixture while reducing negative side effects due to e.g. constant exposure at said dose. Likewise, it may be beneficial to only provide the gaseous mixture at (varying) breathing intervals, to e.g. allow the gaseous mixture to cause an effect and allow the patient with lower toxicity or a lower burden due to the application of the gaseous mixture. This may also be combined with the application in at least a pulse throughout an inhalation cycle, for example, by applying multiple short high dose bursts to individual breaths, e.g. one in every 2 - 30 breaths. Application of a constant concentration throughout the inhalation cycle may be provided to e.g. ensure application throughout the entire respiratory system and increase duration of exposure to the gaseous mixture to increase efficacy.

Furthermore, the controller may be programmed to cause the gaseous mixture to be provided at a variable dose. For example, a treatment regimen may comprise the application of high and low doses that are applied at different time points for e.g. optimal eradication of a biofilm present in the respiratory system of the patient. Furthermore, when implementing an algorithm to cause a target cumulative dose of the gaseous mixture to be applied, the dose level or application may also be set to be dynamically adjusted by the algorithm. This at least has the advantage that an optimal balance between, e.g., bacterial suppression, secretion clearance, and symptom control may be achieved. The variable dose may further be patient-specific, e.g. based on a physical condition of the patient. For example, when the patient is in good physical condition, the treatment regimen may e.g. comprise more high-dose applications of the gaseous mixture compared with patients that are in poor physical condition.

According to another aspect of the invention, the application device may comprise separated channels or lumens for nitric oxide and oxygen and/or air. This minimizes the formation of e.g. NO₂ in the delivery system and hence reduces the formation of unwanted toxic by-products. For example, nitric oxide and oxygen may be provided from the source throughout the entire application device in separate channels until they reach the patient interface. Preferably, the components of the gaseous mixture are mixed preferably just before they exit the patient interface by appropriate means, e.g. by a gas mixer. Alternatively, nitric oxide, oxygen, and air may also exit the patient interface in parallel, without mixing. Since oxygen is generally not reactive with air, only a separate lumen for nitric oxide may be provided while oxygen and air may be combined in another lumen.

The device according to the invention may be applied to a variety of respiratory disorders or complications thereof, in particular one of a ventilator associated pneumonia (VAP), a toxoplasmosis, a heparin-protamine reaction, a traumatic injury, a traumatic injury to the respiratory tract, acidosis or sepsis, acute mountain sickness, acute pulmonary edema, acute pulmonary hypertension, acute pulmonary thromboembolism, adult respiratory distress syndrome, an acute pulmonary vasoconstriction, aspiration or inhalation injury or poisoning, asthma or status asthmaticus, bronchopulmonary dysplasia, hypoxia or chronic hypoxia, chronic pulmonary hypertension, chronic pulmonary thromboembolism, cystic fibrosis (CF), fat embolism of the lung, haline membrane disease, idiopathic or primary pulmonary hypertension, inflammation of the lung, perinatal aspiration syndrome, persistent pulmonary hypertension of a newborn, post cardiac surgery, a bacterial-, viral- and/or fungal bronchiolitis, a bacterial-, viral- and/or fungal pharyngitis and/or laryngotracheitis, a bacterial-, viral- and/or fungal pneumonia, a bacterial-, viral- and/or fungal sinusitis, a bacterial-, viral- and/or fungal upper and/or lower respiratory tract infection, a bacterial-, viral- and/or fungal- exacerbated asthma, a respiratory syncytial viral infection, bronchiectasis, bronchitis, chronic obstructive lung disease (COPD), cystic fibrosis (CF), acute respiratory distress syndrome (ARDS), emphysema, otitis, otitis media, primary ciliary dyskinesia (PCD), and pulmonary aspergillosis (ABPA) and cryptococcosis.

Furthermore, the device may comprise a positive airway pressure device, wherein the controller is programmed to control the positive airway pressure device to provide the gaseous mixture at an adjustable pressure. This may be particularly beneficial for patients with certain respiratory disorders, which are not capable or not sufficiently capable to efficiently breathe. Likewise, the burden of patients that have breathing difficulties may be relieved when applying positive airway pressure. Respiratory muscle fatigue or ventilatory failure can thus be reduced or even prevented and the improved access to the respiratory system may further facilitate mechanical interventions such as e.g. laparoscopic removal, or other simultaneous therapies. The application of positive airway pressure may furthermore have at least the advantage that patients with insufficient respiratory ventilation may e.g. increase the surface area of alveoli that are exposed to the gaseous mixture, thereby increasing the efficacy of the application. Dependent on the configuration, the positive airway pressure device may replace the application device.

According to another aspect of the invention, the device may comprise a user interface in communication with the controller, wherein the user interface is configured to display and/or adjust at least a treatment regimen. The user interface preferably comprises at least an inputting means, e.g. a keyboard, and a display such as a monitor. Preferably, the display and the inputting means may be combined, for example, as a touchscreen. The patient or medical professional may retrieve information regarding the treatment, e.g., the current dose of the gaseous mixture, the application interval and pattern, the set total and current cumulative dose of the gaseous mixture per period of time, patient-specific information, variables included in the algorithm, etc., and may accordingly adjust at least one of the displayed values.

Since medical professionals often treat and/or monitor a plurality of patients simultaneously, it is furthermore beneficial for a medical professional to e.g. monitor the patient and retrieve information regarding its treatment status from a remote location, in particular for home care, as described above. Accordingly, the device may comprise communication means that are in communication with the controller for providing a connection to a remote controller. Such communication means may e.g. comprise transmission and receiving means such as e.g. an antenna to provide radio access and wireless transmission via a shared and/or remote network to a medical professional at a remote location. A medical professional may hence receive the information regarding the current treatment, preferably the information displayed on a user interface, on e.g. a computer system or a hand-held device such as a PDA. Accordingly, the treatment regimen and/or the application of the gaseous mixture may be adjusted. Such a form of telemedicine is in particular beneficial when the patient uses the device as e.g. a portable device and is not located at a medical institution but at e.g. the patient's home, or when the autonomous device is installed at a patient's home. The patient receiving home care may thus be monitored by a medical professional at a remote location and the application and efficacy of the gaseous mixture may be monitored and/or retrieved at any time.

Furthermore, when problems arise during treatment of the patient, a medical professional and/or a technician may be automatically prompted to ensure safety of the patient and continuity of the application. For example, in case of exacerbation of the patient's condition, the device, in particular through an input at the controller provided by the algorithm, may measure and/or recognize anomalies in e.g. the breathing pattern or in the constitution of the exhaled breathing gas. Alternatively or in addition, at least one physiological parameter of the patient may be measured such as, for example, blood oxygen saturation, heart frequency and/or blood pressure. By measuring these parameters the onset of clinical changes such as deterioration (e.g exacerbations) may be determined which may signify the necessity for an intervention or change in therapy. The measurements may also serve to establish future treatment regimes (ADD). Such parameters may be provided by instruments known in the art and are preferably optics-based instruments.

Accordingly, the information may be retrieved by a medical professional by sending the information via the above described communication means and receiving the information at a remote location, e.g., by storing and accessing the information via a cloud-based network. The medical professional may then assess the information and the patient's status and may accordingly adjust the treatment through e.g. control and/or adjustment of the controller. For example, the medical professional may choose to lower the concentration and application interval of nitric oxide and/or increase the concentration of oxygen.

If the patient's condition does not improve within a desired amount of time, e.g. the measured blood oxygen saturation and/or the measured blood pressure fall below or within a predetermined safety range, the device may automatically alarm a medical professional and/or call a paramedic in emergency situations.

The device may furthermore comprise a connection means such as a USB-connection or a serial cable to connect the device with a computer system. Alternatively, such a connection may be provided with the above described communication means. A connection with a computer system allows data regarding the treatment of the patient to be read-out and allows for incorporation into e.g. a database and/or software. The patient-specific treatment data may be compared to optimise future treatment regimens and/or to adjust treatment regimens for the current patient and other patients. Data may furthermore be subject to statistical analysis to e.g. calculate and/or predict efficacy of the current or future treatment regimens.

The device according to the invention may further contemplate that all components mediating gas flow are biocompatible and are inert with the gaseous mixture. Especially for application to a patient's interface this is of essential importance since any occurring gas reaction from a gas source towards the patient interface may produce toxic compounds that are detrimental effects and reduce the patient's safety. For example, all applied tubing are provided from a biocompatible materials and dissolvable coatings that may release and/or expose reactive oxides should be avoided. Exchangeable components may furthermore be chosen out of a list of materials that are furthermore recyclable, durable and/or sustainable. To increase the mobility or portability of the device, the components may furthermore be chosen to be lightweight while providing sufficient robustness.

At least a further advantage of the onsite production of nitric oxide and the onsite enrichment of oxygen is that any desired concentration may be instantly provided. For example, whereas commonly used gas tanks only comprise a single concentration of a gas, the onsite production may provide variable concentrations at any desirable time point. This is much more effective and requires much less steps to achieve the desired concentration when compared with the implementation using common gas tanks, for example, mixing and/or dilution steps may be omitted and the costs associated with production and storage of pure gases, which are otherwise necessary to reach any desirable higher concentration than ambient air, are non-existent. Furthermore, gas quality and/or filling accuracy in common used gas tanks may vary and require frequent and strict quality inspections. The gas quality and concentration may also vary over time since e.g. nitric oxide is highly reactive with oxygen and unwanted side-reactions may take place. By the same token, unwanted particles or compounds may accumulate in gas tanks over time due to frequent filling. The onsite production of nitric oxide and the onsite enrichment of oxygen hence provide a safer and more accurate method to provide nitric oxide and oxygen, respectively, while simultaneously providing more flexibility and reducing costs.

Preferably, the oxygen is present in the gaseous mixture in a concentration of between 10 to 50 percent, preferably between 20 and 30 percent. The oxygen may also be provided using ambient air, as described above. Ambient air generally comprises oxygen levels of 20 to 21 percent and furthermore comprises high levels of nitrogen, a low level of carbon dioxide and negligible levels of other elements. Ambient air can thus be used to provide the gaseous mixture with a sufficient oxygen level. If higher oxygen doses are desired the gaseous mixture can furthermore be enriched by oxygen using the arrangement for onsite enrichment of oxygen. This has e.g. the advantage that lower amounts of enriched or pure oxygen are required, reducing potential wear, noise, and/or energy consumption of the respective arrangement.

Preferably, the nitric oxide is present in the gaseous mixture in a concentration of between 0.1 ppm and 1000 ppm, preferably between 10 ppm and 250 ppm, more preferred between 10 ppm and 20 ppm or between 80 ppm and 160 ppm or between 160 ppm and 250 ppm. Accordingly, the concentrations of the nitric oxide may be very low but a continuous application thereof prevents or at least reduces the multiplication of bacteria, viruses and fungi by slowing down or even supressing growth. Furthermore, continuous application of low concentrations of nitric oxide may cause dispersal of a biofilm, thereby improving e.g. the breathing efficiency and/or facilitates proper clearance of the patient's airways. When higher concentrations of the ranges given above are used, bacteria, viruses and fungi can be killed, biofilm formation can be reduced and/or prevented, and blood flow and oxygenation is improved.

The configuration of the device may furthermore allow that the gaseous mixture to be applied comprises other components in addition to nitric oxide, oxygen, and/or air. For example, the gaseous mixture may comprise a medicament or another gas such as helium for the treatment of a respiratory disorder or the treatment of complications thereof. Accordingly, the device may comprise another injector that is at least in communication with the controller and the application device. Preferably, the injector comprises a nebulizer in the case of a medicament. Such a nebulizer can either be in direct communication with the injector or can be provided as an autonomous injector that replaces said injector. Alternatively, the injector comprises a dry powder inhaler in the case of a medicament. Such an inhaler can likewise be either in direct communication with the injector or can be provided as an autonomous injector that replaces said injector. The injection may be provided continuously, e.g. in very low doses, but preferably occurs at predetermined intervals and with predetermined frequencies and/or pulses, or depending on the algorithm, as described above. The provision of another medicament or gas can also be performed by an existing gas injector by provision of e.g. a second reservoir and/or a mixing and dispensing chamber.

According to another embodiment of the present invention, the apparatus comprises means to measure flow characteristics of the exhaled breathing gas. Flow characteristics may be measured to provide information such as e.g. breathing resistance, turbulence, or breathing volume, which can provide an indication of potential obstructions and/or the efficacy of the therapy. Such means are also known in the art, such as e.g. flowrate sensors.

The above means for measuring molecular concentrations present in the exhaled breathing gas and the means to measure flow characteristics of the exhaled breathing gas may be combined in a single means, such as e.g. a single sensor. Furthermore, the device may comprise a separate controller or a control unit within a common controller for processing the acquired data. As such, the therapy may be adjusted accordingly.

Instead of filtering out humidity, the device may furthermore add a level of humidity to the gaseous mixture to be applied to counteract the reduced humidification found in many patients. Humidification may improve the clearance and hence counteract the reduced cough reflex and improves extravasation and infiltration of the immune system into the pathogenic area. Accordingly, the device may comprise an humidifier. Humidifiers are known in the art and are based on e.g. a nebulizer and a sterile water or saline containing reservoir.

Furthermore, the apparatus may comprise a safety means, in case the acquired data exceed predetermined thresholds. Such a safety means may e.g. comprise an emergency stop of the application or transmission of an emergency signal, when e.g. concentrations measured in the exhaled breathing gas indicate the occurrence of complications. For example, if a patient stops breathing for a certain period of time or the volume or consistency of the exhaled breathing gas are distinct from predefined values, e.g. indicate increased acidosis, an alarm may be triggered and/or the level of e.g. oxygen and/or nitric oxide may be adjusted accordingly. A similar mechanism may allow to check whether the gaseous mixture to be applied is properly applied and inhaled, e.g., by providing feedback of a flow rate sensor and/or an exhaled breathing gas concentration. The patient may then be notified. This notification may be haptic, acoustic, and/or visual. In case no change is measured after a predetermined period of time, the device may trigger an alarm and/or a medical professional may be notified, preferably automatically. As described above, the safety means may also comprise an input for a measurement of at least one physiological parameter of the patient such as e.g. an indicator of blood oxygenation or blood pressure. This information may also be provided as an input for the algorithm and/or controller. The safety means may alarm the medical professional, who may then assess the information and the patient's status and may accordingly adjust the treatment through e.g. control and/or adjustment of the controller.

### Brief description of the drawings

The present disclosure will be more readily appreciated by reference to the following detailed description when being considered in connection with the accompanying drawings in which:
Figure 1 is a schematic view of a device for providing a gaseous mixture to a patient; and
Figure 2 is a schematic view of another device for providing a gaseous mixture to a patient.

In the following, the invention will be explained in more detail with reference to the accompanying Figures. In the Figures, like elements are denoted by identical reference numerals and repeated description thereof may be omitted in order to avoid redundancies.

### Detailed description of preferred embodiments

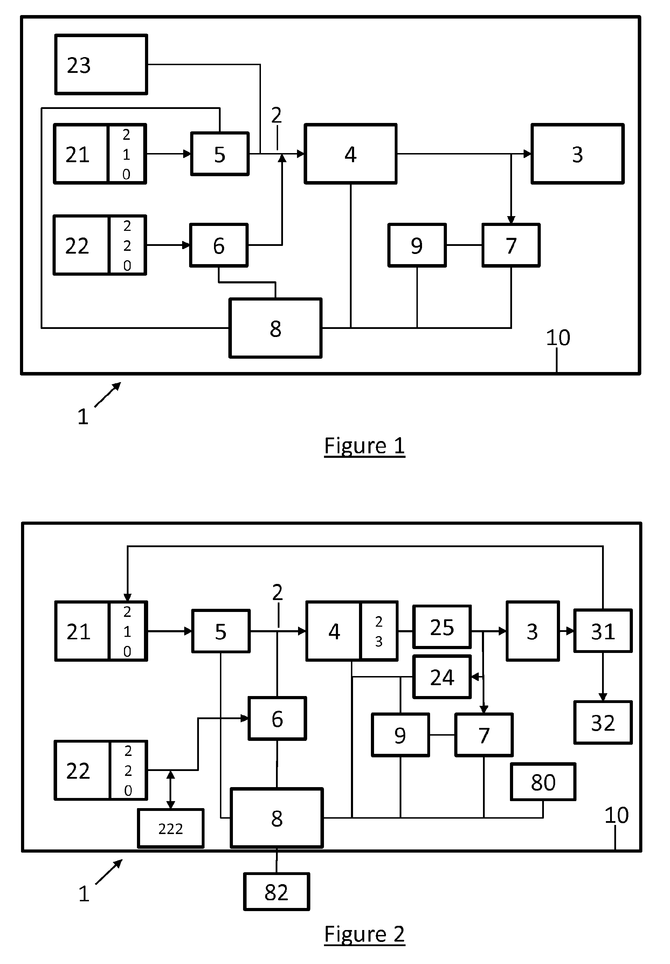

In Figure 1 a device for providing a gaseous mixture to a patient is shown, as indicated by 1. A gaseous mixture 2 is provided to an application device and originates from a source of air 23, a source of gaseous nitric oxide 21 and a source of gaseous oxygen 22, wherein the source of gaseous nitric oxide 21 comprises an arrangement for onsite production of nitric oxide 210 and the source of gaseous oxygen 22 comprises an arrangement for onsite enrichment of oxygen 220. An application device 4 can be any device for the delivery of a gaseous mixture as known in the art. Although the arrangement for onsite production of nitric oxide 210 and the arrangement for onsite enrichment of oxygen 220 are depicted to be comprised within the source of gaseous nitric oxide 21 and a source of gaseous oxygen 22, this is only for the purpose of overview.

The source of air 23 may be provided by ambient air. Preferably, the source of air 23 comprises a filter arrangement (not shown) for providing air without pollutants. Accordingly, potential toxic chemical compounds such as carbon monoxide, ozone, sulfur dioxide, dust, and/or particulate matter, may be filtered out. In addition, the filter arrangement may be configured to prevent pathogens to enter the device and consequently enter the respiratory system of the patient. Pathogens such as e.g. viruses and/or airborne bacteria may hence be filtered out. Accordingly, the source of air 23 may comprise a filter arrangement for providing sterile medical grade air. Filters with a variety of mechanisms may be provided, e.g., chemical-based, mechanical, ionic binding-based, absorption-based, electromagnetic, etc., known in the art. To ensure appropriate flow to the application device 4, the source of air 23 may furthermore comprise e.g. a pumping device or compressor (not shown). Alternatively, the source of air 23 may be integrated into the application device 4.

Arrangements and methods for onsite nitric oxide production are known in the field and allow the generation of NO through nitrogen and oxygen present in ambient air by for example a pulsating electrical discharge or an electric arc, see e.g. WO 2013/05248 A2 and WO 2014/143842 A1, respectively. The methods described here should not be appreciated such that these are limiting, but merely provide examples from a plurality of alternative methods known in the art.

Arrangements and methods for onsite oxygen enrichment are known in the art. For example, pressure swing adsorption-based arrangements (PSA) may be chosen, or, in particular for portable configurations, rapid PSA arrangements may be preferred.

The components of the device 1 are preferably comprised within a single housing 10, as depicted in Figure 1. Preferably, the device 1 is configured to be portable and may furthermore function autonomously by implementing a rechargeable electrical energy storage device such as a battery (not shown).

The arrangement for onsite production of nitric oxide and the arrangement for onsite enrichment of oxygen may produce nitric oxide and oxygen, respectively, using ambient air. It may further be desired and/or required to implement air filters when using ambient air to prevent potential infiltration of e.g. aerosol pathogens present in the ambient air into the respiratory system (not shown).

The injection of nitric oxide occurs via a gas injector 5 into the gaseous mixture 2 while the injection of oxygen occurs via a gas injector 6 into the gaseous mixture 2. Although Figure 1 shows that the injection of both nitric oxide and oxygen are performed before the application device 4, one of the gases may also be injected at a point in the gaseous mixture 2 at or after the application device 4, if configured accordingly. In case nitric oxide and oxygen are only added to enrich ambient air, both gases may also be injected after the application device 4.

The gaseous mixture 2 enriched with nitric oxide and oxygen is then provided at the downstream patient interface 3 to be inhaled as a medicament by the patient.

Furthermore, the device 1 is provided with a controller 8 to control the injection of the gas injectors 5 and. In addition, the controller 8 controls the application device 4 for the provision of the gaseous mixture 2 and can do so depending on gas flow measurements provided by the flow rate sensor 7. For example, upon initiation of an inhalation phase by the patient, gas flow through the patient interface 3 can cause a pressure decrease and a flow rate to be measured by the flow rate sensor 7. When the controller 8 receives this information it can control the injection of nitric oxide and oxygen while providing the gaseous mixture 2 by actuating the application device 4.

By the same token, an exhalation phase may cause a pressure increase through the patient interface 3, which causes the flow rate sensor 7 to detect an exhalation phase. As soon as the exhalation phase is terminated, the corresponding loss of pressure may also provide the controller 8 with measurements that cause the application device 4 to provide the gaseous mixture 2, with or without further injection of nitric oxide and/or oxygen. In doing so, the provision of the gaseous mixture 2 may occur before the inhalation phase is initiated by the patient, which further reduces the breathing effort and facilitates an improvement in pulmonary gas influx. By the same token, the arrangement for onsite production of nitric oxide 210 and the arrangement for onsite enrichment of oxygen 220 may generate the respective gas based on a flow measured by the flow rate sensor 7, e.g., upon inhalation or during an exhalation phase in order to timely coordinate the respective injection and/or application.

As depicted in Figure 1 the device may furthermore comprise a processing means 9. The processing means is in communication with at least a flow rate sensor 7 and the controller 8. The processing means uses an algorithm to apply the gaseous mixture 2 at a target cumulative dose and provides the controller with an input to control the gas injectors 5, 6, and the application device 4. By the same token, the algorithm may adjust the output after calculation of input provided by e.g. the flow rate sensor 7 and/or the controller 8. The arrangement for onsite production of nitric oxide 210 and the arrangement for onsite enrichment of oxygen 220 may be activated by activation of the respective gas injector 5, 6 or directly by the controller 8 (not shown).

The device may further comprise an in-line heating or cooling unit (not shown) to provide the gaseous mixture at a desired and/or patient specific temperature.

In Figure 2 another exemplary embodiment of a device for providing a gaseous mixture 2 to a patient according to the present invention is shown, as indicated by 1. In addition to the device 1 from Figure 1, a storage reservoir 222 for oxygen is provided. Although the reservoir 222 are is depicted to be comprised outside of the source of gaseous oxygen 22, this is only for the purpose of overview, i.e. they may also be comprised within the means 22 in an alternative configuration. Exemplary only, the source of air 23 is alternatively depicted to be part of the application device 4. The device may furthermore comprise a filter 25 before or at the patient interface 3 to filter out potentially toxic components such as, e.g., nitrogen oxide.

Furthermore, a sensor arrangement 24 is shown that is in direct communication with the gaseous mixture 2 and provides the controller 8 and/or the processing means 9 with input regarding a characteristic of the gaseous mixture 2, e.g. a concentration of nitric oxide present in the gaseous mixture 2. Preferably, as shown in Figure 2, the sensor arrangement 24 is in line with the flow rate sensor 7 to both minimize the size of the device 1 and to reduce the amount of gas lost. However, other embodiments are possible, wherein these measurements are performed in parallel. The measured values are processed by the controller 8, which is in communication with the sensor arrangement 24, 7 and which can accordingly adjust the injection of nitric oxide and/or oxide, and/or the total application of the gaseous mixture 2.

From the patient interface 3 a filter 31 for the exhaled breathing gas is furthermore provided. The filter 31 may selectively filter out a component of the exhaled breathing gas. This filtered out gas may then be redistributed to the arrangement for onsite production of nitric oxide 210 (shown) and/or another gas to the arrangement for onsite enrichment of oxygen 220 (not shown). The remaining gas that may be disposed of is conducted to a reservoir 32 to prevent leakage to the environment and/or ambient air.

The filter 31 may also comprise a gas and/or chemical sensor (not shown) that is in communication with the controller 8 (not shown), which can accordingly adjust the injection of nitric oxide, oxygen, and/or the total application of the gaseous mixture 2 to adjust the medical treatment and/or prevent the unwanted *in vivo* accumulation of potentially toxic compounds.

As depicted in Figure 2, the controller 8 may furthermore comprise a user interface 82 to both display and input of treatment values. The user interface 82 is depicted outside of the housing 10, however may also be integrated at its surface or may be wirelessly connected to the device 8, in particular the controller 8, e.g. via a communication means 80. The communications means may furthermore allow external access from e.g. a medical professional to the device to check, monitor, and/or adjust the application settings for the treatment in the controller and/or processing means.

Although not depicted in figures 1 or 2, sensor arrangements may also be provided in other components such as the reservoir 222 or at the arrangement for onsite production of nitric oxide 210 and/or the arrangement for onsite enrichment of oxygen 220. Such sensors may further optimize the feedback provided at the controller 8 and/or the processing means 9. In particular in the latter case, the algorithm may further adjust the application of the gaseous mixture to achieve a target cumulative dose of the gaseous mixture according to the obtained physiological data of the patient and by providing a corresponding output to the controller 8.

For all embodiments, the device 1 may be programmed for different, preferably patient-specific, treatment regimens. For example, a treatment regimen may provide e.g. a nitric oxide delivery peak in the morning to cleanse the lungs after sleeping and/or before the patient falls asleep in the evening. Alternatively, or in addition, the target cumulative dose may vary between 24 hour periods, e.g., allowing for days off therapy or intensive therapy, when the patient, e.g., copes with an infection, or when treatment may cause deteriorating symptoms in the patient. Furthermore, the target cumulative dose may vary as part of a multiday treatment schedule.

The controller 8 may be programmed to cause the gaseous mixture 2 to be provided continuously, intermittently, and/or at a predetermined time interval. Accordingly, the target cumulative dose, e.g. of nitric oxide, may also be applied by varying the application within a 24 hour period, between days, and/or weeks. For example, a static dose level may be continuously applied to provide a form of background therapy or prophylaxis at a continuous low dose. Alternatively, intermittent application may be preferred, so that the device 1 applies the gaseous mixture at a static dose level at different time points, for example, as part of a resistant bacteria eradication protocol, or for shorter durations, e.g. for more active patients receiving oxygen co-therapy. When providing intermittent application, the patient may breathe non-enriched, preferably sterile, air provided by the device or, alternatively, may breathe ambient air during recovery periods. However, for both intermittent and continuous application the target cumulative dose preferably does not change. When applying the gaseous mixture for larger time periods, the target cumulative dose may be varied on e.g. a daily or weekly base. For example, nitric oxide may be applied during a first week of treatment while being absent or being present at a lower dose in the gaseous mixture during application in subsequent weeks. This allows weaning of a patient, i.e. gradual reduction of the treatment and hence a gradual increase in autonomous breathing of the patient while preventing e.g. the occurrence of a respiratory infection or a relapse thereof. This is in particular beneficial for patients suffering from COPD.

The application of the gaseous mixture 2 may also occur as a combination of a continuous and an intermittent application. For example, a continuous low dose of nitric oxide may be applied to a patient while a higher dose of nitric oxide may be intermittently applied. The intermittent application may be provided by multiple short high dose bursts lasting e.g. for 10 seconds up to 30 minutes, but may also last longer, e.g. be varied on a daily basis, as described above. The duration time of these bursts may be dependent on the therapeutic goal to be achieved, e.g., minimize coughing, respiratory irritation, and other symptoms. Preferably, the time function may be adjustable according to the algorithm and may hence vary not only per patient but also with an altered physiological state of the patient. The combination of continuous and intermittent application may be particularly beneficial over night, when symptoms that may produce sleep disturbance are to be reduced.

When applying intermittent dosing, not only shorter treatment durations may be chosen, but the dose to be applied may also be higher, when compared with e.g. continuous application or background therapy, as described above. Application of a high dose of e.g. nitric oxide in the evening before bed time may expectorate secretions, cause microbial kill, and/or suppress overnight bacterial regrowth. Simultaneously, application of a high dose of e.g. nitric oxide in the morning expectorates secretions/microbes that have accumulated overnight so that the microbial and/or secretion load is reduced throughout the day, enabling e.g. more effective respiratory physiotherapy, higher activity, and/or improved delivery of inhaled medicaments for the patient. The high and low doses of the gaseous mixture may also be combined with different treatment regimens, as, for example, described above.

The controller 8 may further be programmed to apply the gaseous mixture 2 with a breath by breath variability and/or with a predetermined breathing frequency. The application may hence also be provided with e.g. a pre-programmed breathing frequency, which is independent of the measurements provided by a flow rate sensor 7. For example, the patient may receive the application with a prescribed breathing frequency and may be, actively or passively, required to adjust his/her breathing frequency accordingly. Alternatively, or additionally, the application may be provided variable breath by breath, e.g., the controller 8 may be programmed to apply the gaseous mixture 2 with a prescribed variability per breath or may provide the application of the gaseous mixture 2 in an on-demand fashion, as described above. Preferably, the controller 8 is provided with input from a flow rate sensor 8 and/or from an output of an algorithm to accordingly provide a breath by breath variability and/or a predetermined breathing frequency.

In addition, the controller 8 may be programmed to control the application of the gaseous mixture 2 in a constant concentration throughout the inhalation cycle, in at least a pulse throughout an inhalation cycle, during every second breathing cycle or during every other natural number of breathing cycles except one. For example, application of the gaseous mixture 2 may be more beneficial during a specific breathing phase, e.g. at the beginning of the inspiratory breath for optimal spreading throughout the respiratory system, or when bracheoli and alveoli of the respiratory system are in their most accessible or widened state, so that e.g. a higher dose of the gaseous mixture 2 may be chosen when applying the gaseous mixture in at least a pulse throughout the respective phase. This at least has the advantage of increasing efficacy of the gaseous mixture 2 while reducing negative side effects due to e.g. constant exposure at said dose. Likewise, it may be beneficial to only provide the gaseous mixture 2 at (varying) breathing intervals, to e.g. allow the gaseous mixture 2 to cause an effect and allow the patient with lower toxicity or a lower burden due to the application of the gaseous mixture2 . This may also be combined with the application in at least a pulse throughout an inhalation cycle, for example, by applying multiple short high dose bursts to individual breaths, e.g. one in every 2 - 30 breaths. Application of a constant concentration throughout the inhalation cycle may be provided to e.g. ensure application throughout the entire respiratory system and increase duration of exposure to the gaseous mixture 2 to increase efficacy.

Furthermore, the controller 8 may be programmed to cause the gaseous mixture 2 to be provided at a variable dose. For example, a treatment regimen may comprise the application of high and low doses that are applied at different time points for e.g. optimal eradication of a biofilm present in the respiratory system of the patient. Furthermore, when implementing an algorithm to cause a target cumulative dose of the gaseous mixture 2 to be applied, the dose level or application may also be set to be dynamically adjusted by the algorithm. This at least has the advantage that an optimal balance between, e.g., bacterial suppression, secretion clearance, and symptom control may be achieved. The variable dose may further be patient-specific, e.g. based on a physical condition of the patient. For example, when the patient is in good physical condition, the treatment regimen may e.g. comprise more high-dose applications of the gaseous mixture 2 compared with patients that are in poor physical condition.

It will be obvious for a person skilled in the art that these embodiments and items only depict examples of a plurality of possibilities. Hence, the embodiments shown here should not be understood to form a limitation of these features and configurations. Any possible combination and configuration of the described features can be chosen according to the scope of the invention.

### List of reference numerals

- 1: Device for providing a gaseous mixture to a patient
- 10: Housing
- 2: Gaseous mixture
- 21: Source of gaseous nitric oxide
- 210: Arrangement for onsite production of nitric oxide
- 22: Source of gaseous oxygen
- 220: Arrangement for onsite enrichment of oxygen
- 222: Storage reservoir for oxygen
- 23: Source of air
- 24: Sensor arrangement
- 25: Filter
- 3: Patient interface
- 31: Filter
- 32: Reservoir
- 4: Application device
- 5: Gas injector for injecting nitric oxide
- 6: Gas injector for injecting oxygen
- 7: Flow rate sensor
- 8: Controller
- 80: Communications means
- 82: User interface
- 9: Processing means

## Claims

1. Device for the treatment of respiratory disorders or complications thereof in a mammal with a gaseous mixture for use as an inhalable medicament, comprising:
a patient interface,
a source of air,
a source of gaseous nitric oxide,
a source of gaseous oxygen,
an application device for providing a gaseous mixture to a patient interface,
at least one gas injector for injecting nitric oxide provided by the source of gaseous nitric oxide into the gaseous mixture provided by the application device,
at least one gas injector for injecting oxygen provided by the source of gaseous oxygen into the gaseous mixture provided by the application device, and
a controller programmed for controlling the at least one gas injector and the application device,
**characterized in that**
the source of gaseous nitric oxide comprises an arrangement for onsite production of nitric oxide, and
the source of gaseous oxygen comprises an arrangement for onsite enrichment of oxygen.

2. Device according to claim 1, wherein the components are provided within a single housing.

3. Device according to claim 1 or 2, wherein it is configured to be portable.

4. Device according to any one of the preceding claims, wherein it comprises at least one rechargeable electrical energy storage device, and wherein the device is configured to operate as a stand-alone device.

5. Device according to any one of the preceding claims, wherein the controller comprises at least one processing means for processing of an algorithm to cause a target cumulative dose of the gaseous mixture to be applied by the at least one gas injector and the application device.

6. Device according to claim 5, wherein the target cumulative dose of the gaseous mixture is defined by a target cumulative dose of nitric oxide.

7. Device according to any one of the preceding claims, wherein it comprises a sensor arrangement for the detection of the concentration of at least one component of the gaseous mixture and which is in communication with the controller.

8. Device according to any one of the preceding claims, wherein it comprises a sensor arrangement for the detection of the concentration of at least one component of exhaled breathing gases, the sensor arrangement being in communication with the controller.

9. Device according to any one of the preceding claims, wherein the controller is programmed to cause the gaseous mixture to be provided continuously, intermittently, and/or at a predetermined time interval.

10. Device according to any one of the preceding claims, wherein it comprises a flow rate sensor which is in communication with the controller.

11. Device according to claim 10, wherein the controller is programmed to apply the gaseous mixture with a breath by breath variability and/or with a predetermined breathing frequency.

12. Device according to any one of the preceding claims, wherein the controller is programmed to control the application of the gaseous mixture in a constant concentration throughout the inhalation cycle, in at least a pulse throughout an inhalation cycle, during every second breathing cycle or during every other natural number of breathing cycles except one.

13. Device according to any one of the preceding claims, wherein the controller is programmed to cause the gaseous mixture to be provided at a variable dose.

14. Device according to any one of the preceding claims, wherein the application device comprises separated channels or lumens for nitric oxide and oxygen.

15. Device according to any one of the preceding claims, wherein it comprises a positive airway pressure device and wherein the controller is programmed to control the positive airway pressure device to provide the gaseous mixture at an adjustable pressure.
